(19) Europäisches Patentamt
European Patent Office
Office européen des brevets

(11) **EP 3 243 481 A1**

(12) **EUROPÄISCHE PATENTANMELDUNG**

(43) Veröffentlichungstag:
15.11.2017 Patentblatt 2017/46

(51) Int Cl.:
*A61C 9/00* *(2006.01)*

(21) Anmeldenummer: 16169206.6

(22) Anmeldetag: 11.05.2016

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(71) Anmelder: **Vita Zahnfabrik H. Rauter GmbH & Co. KG**
**79713 Bad Säckingen (DE)**

(72) Erfinder:
• **CHRISTEN, Urban**
**79713 Bad Säckingen (DE)**
• **KERSCHENSTEINER, Eva**
**79713 Bad Säckingen (DE)**
• **EGLE, Franz**
**79713 Bad Säckingen (DE)**

(74) Vertreter: **dompatent von Kreisler Selting Werner - Partnerschaft von Patent- und Rechtsanwälten mbB**
**Deichmannhaus am Dom**
**Bahnhofsvorplatz 1**
**50667 Köln (DE)**

(54) **VERFAHREN ZUR FESTLEGUNG EINER ZAHNAUFSTELLUNG**

(57) Verfahren zur Festlegung einer Zahnaufstellung für die Herstellung von Zahnteil- oder Vollprothese mit den Schritten: Bestimmen von durch Koordinaten definierte Basispunkte eines Unterkiefers und/oder einer Oberkiefers; Ermitteln eines Transversalmaßes und/oder einer skelettalen Klasse und/oder einer Bissart auf Basis der bestimmten Koordinaten für den Unterkiefer und/oder den Oberkiefer und Auswahl einer passenden Zahnaufstellung auf Grundlage des ermittelten Transversalmaßes und/oder der ermittelten skelettalen Klasse und/oder der ermittelten Bissart.

| 1.Okklusionskonzept | ⇨ | manuelle Auswahl | ⇨ | Bukkal, Ligualisiert, Gerber, Grunert |
| 2.Garniturmaß | ⇨ | manuelle Eingabe der Nasenbreit | ⇨ | Bestimmung durch Funktions-Generik |
| 3.Transversalmaß | ⇨ | manuelle Eingabe von Landmarks | ⇨ | Bestimmung durch Funktions-Generik |
| 4.Skeletale Klasse | ⇨ | manuelle Eingabe von Landmarks | ⇨ | Bestimmung durch Funktions-Generik |
| 5.Biss Art | ⇨ | manuelle Eingabe von Landmarks | ⇨ | Bestimmung durch Funktions-Generik |
| 6.Zahnlinie | ⇨ | manuelle Auswahl | ⇨ | VITAPAN PLUS, LINGOFORM, VIONIC |
| 7.Zahnform | ⇨ | manuelle Auswahl | ⇨ | R, O, T |

Ziel: Anwender wählt aus maximal 2-5 verbleibenden Aufstellungen

**Fig.2**

**Beschreibung**

Die Erfindung betrifft ein Verfahren zur Festlegung einer Zahnaufstellung für die Herstellung von Zahnvoll- und/oder Zahnteilprothesen.

Bei der Versorgung von Patienten mit dentalen Teil- oder Vollprothesen ist heutzutage ein hoher Standard festzustellen. Der Zahnarzt bereitet die zur Versorgung vorgesehene Situation am Patienten vor, während die Zahn(teil)prothese üblicherweise in einem externen Labor oder einem Praxislabor von einem Zahntechniker nach den Vorgaben des Zahnarztes hergestellt wird. Die Qualität der Prothese ist dabei in großem Maße abhängig von der handwerklichen Geschicklichkeit des Zahntechnikers, der die Vorgaben des Zahnarztes in der Herstellung der Prothese berücksichtigen muss, sowie der Qualität dieser Vorgaben.

Des Weiteren ist die Vorbereitung des prothetisch zu versorgenden Patienten für diesen oftmals mit Unannehmlichkeiten verbunden. Erwähnt seien in diesem Zusammenhang beispielsweise verschiedene Sitzungen beim behandelnden Zahnarzt, in denen Abformungen der zu versorgenden Situation vorgenommen werden müssen, die dem Zahntechniker als Negativmodelle dienen. Die sich anschließenden weiteren Abformungen sind zeitaufwendig und oftmals der Grund für fehlerhafte Ausführung der prothetischen Versorgung. Typischerweise sind zwei oder drei Abformungssitzungen erforderlich, bevor der Zahntechniker mit der eigentlichen Herstellung der Prothese beginnen kann.

WO 2011/066895 A1 betrifft ein Verfahren für die automatisierte Herstellung von Zahnersatz, umfassend die Schritte Bereitstellen eines digitalen Datensatzes der herzustellenden individuellen Prothese, digitale Trennung des Modells in Zahnbogen und Zahnfleisch, Herstellung des Zahnbogens aus Keramik und Kunststoff mittels Frästechnik oder Herstellung der Prothesenbasis durch generative oder abtragende Methoden aus überwiegend (Meth)-Acrylat-basierten Kunststoffen, den Anschluss des Zahnbogens und das Zahnfleisch durch Kleben oder Verbinden oder eine Kombination von Kleben und Fügen.

EP 1 864 627 A2 offenbart ein Verfahren zur Herstellung von Zahnprothesen nach einem digitalisierten, virtuellen Modell, welches die Kiefersituation wiedergibt, mit den Schritten einer digitalen Erfassung der Kiefersituation und -relationen, digitalen Aufstellung (automatisch als Option), Erzeugung einer geteilten Negativform (Rapid Manufacturing) aus den Daten der digitalen Zahnaufstellung, Einstecken der Konfektionszähne/ konfektionierten Zahneinheiten in die geöffneten Negativformen, Schließen der Negativformen, und Ausfüllen der verbliebenen Hohlräume mit Prothesenkunststoff.

EP 1 444 965 A2 betrifft ein Verfahren zur Herstellung von Zahnersatz, mit den Schritten, Aufnahme und Digitalisierung (Einscannen) der 3-dimensionalen, anatomischen Verhältnisse in der Mundhöhle, gegebenenfalls Aufnahme und Digitalisierung (Einscannen) der 3-dimensionalen Daten von Bissschablonen incl. Bisswällen, gegebenenfalls Aufnahme der Kieferdaten, die normalerweise am Patienten zur Einstellung des Artikulators genommen werden, erhaltenen Verarbeitung des Datensatzes in der Weise, dass die relevanten anatomischen Strukturen für eine virtuelle Zahnaufstellung festliegen und ein virtuelles Modell als Datensatz erhalten wird, gefolgt von der Auswahl der 3-D Datensätze konfektionierter, vorher eingescannter Zähne aus einem weiteren Datensatz, virtuelle Aufstellung der Zähne in das virtuelle Modell als zweiten Datensatz sowie entweder gefolgt von einem Übertragen der virtuellen Aufstellung auf das Modell durch entweder Positionierschablone (z.B. gefräst oder Rapid Prototyped) oder direkte Aufstellung der konfektionierten Zähne auf das Modell, Fixierung der Zähne auf dem Modell, Anbringen der Prothesenbasis oder in einer anderen Alternative, gefolgt von einer direkten Herstellung der Prothesenbasis - nach den Daten der virtuellen Zahnaufstellung - mit Positionierhilfen für die endgültige korrekte Positionierung und Fixierung der konfektionierten Zähne. Dieses Verfahren zur Herstellung von Zahnersatz ist äußerst aufwendig. Insbesondere ist der Aufwand hoch um einen präzisen Zahnersatz herzustellen.

Aufgabe der Erfindung ist es, ein Verfahren zur Festlegung einer Zahnaufstellung für die Herstellung von Zahnvollprothesen oder Teilprothesen zu schaffen, mit dem auf möglichst einfache Weise eine zufriedenstellende Auswahl der Zahnaufstellung erfolgen kann.

Die Lösung der Aufgabe erfolgt erfindungsgemäß durch die Merkmale des Anspruchs 1.

Zunächst erfolgt das Erfassen der oralen Patientensituation. Dies kann durch bekannte Verfahren beispielsweise durch Abformen und Bissnahme und/oder mittels digitaler Aufnahmen und gegebenenfalls Digitalisierung der oralen Patientensituation erfolgen. Beispielsweise beim Verwenden von Bissschablonen für den Ober- und den Unterkiefer erfolgt ein Abdruck der aktuellen Patientensituation. Diese kann digitalisiert werden, wobei das Negativ der Bissschablone die tatsächliche orale Patientensituation abbildet, so dass diese im Rechner abgebildet ist. Eine entsprechende Abbildung der oralen Patientensituation kann auch durch unmittelbares digitales Abtasten der Patientensituation erfolgen. Als Ergebnis ist stets die tatsächliche orale Patientensituation im Rechner abgebildet. Erfindungsgemäß erfolgt zunächst ein Bestimmen von Koordinaten definierter Basispunkte eines Unter- und/oder eines Oberkiefers. Dies erfolgt vorzugsweise mittels des im Rechner abgebildeten Unterkiefers- und/oder Oberkiefers, das heißt der abgebildeten oralen Patientensituation.

Auf Grundlage der koordinatendefinierten Basispunkte ist es möglich, für den Unterkiefer und/oder den Oberkiefer ein Transversalmaß und/oder eine skelettale Klasse und/oder eine Bissart zu ermitteln. Hierbei ist es insbesondere bevorzugt, dass insbesondere zwei und besonders bevorzugt drei dieser Merkmale möglichst detailliert bestimmt werden. Je mehr Merkmale und je detaillierter diese Merkmale bestimmt werden, desto qualitativ hochwertiger ist die Festlegung der Zahnaufstellung.

Im nächsten Schritt erfolgt sodann eine Auswahl einer passenden Zahnaufstellung auf Grundlage der ermittelten Daten, das heißt auf Grundlage des ermittelten Transversalmaßes und/oder der ermittelten skelettalen Klasse und/oder der ermittelten Bissart.

Die ermittelte Zahnaufstellung und somit die festgelegten künstlichen Zähne werden sodann in einem weiteren Schritt in eine angefertigte Prothesenbasis eingesetzt. Die Herstellung der Prothesenbasis kann hierbei beispielsweise durch ein CAD/CAM-System wie in WO 2015/078701 beschrieben erfolgen.

In besonders bevorzugter Ausführungsform der Erfindung erfolgt das Bestimmen des Transversalmaßes als Abstand der tiefsten Stellen des Kieferkamms. Dies erfolgt vorzugsweise aufgrund der im Rechner abgebildeten tatsächlichen oralen Patientensituation des Ober- und/oder Unterkiefers. Hierzu ist es insbesondere bevorzugt, dass die Basispunkte die am weitesten mesial gelegenen Punkte der retromolaren Dreiecke auf der rechten und linken Seite des Unterkiefers umfassen. Das Transversalmaß kann sodann vorzugsweise aus dem Abstand zwischen den beiden am weitesten mesial gelegenen Punkten erfolgen.

Das Transversalmaß kann somit vorzugsweise ermittelt werden durch

$$\frac{rd - tmm}{2} = y$$

wobei

$t_{mm}$ das Transversalmaß

$r_d$ die Strecken zwischen den mesialen Punkten des retromolaren Dreiecks und y der Umrechnungsfaktor ist.

In bevorzugter Ausführungsform werden die Koordinaten der entsprechenden Basispunkte, das heißt zur Ermittlung des Transversalmaßes die Koordinaten der beiden mesialsten Punkte der retromolaren Dreiecke ermittelt. Der Abstand $d_r$ berechnet sich bei der Verwendung von kartesischen Koordinaten aus

$$d_r(rdr;\ rdl) = \sqrt{(x_{rdr} + x_{rdl})^2 + (y_{rdr} - y_{rdl})^2 + (z_{rdr} - z_{rdl})^2} \quad \text{(Gl. 2)}$$

wobei x, y, z, die Koordinaten des linken (l) bzw. des rechten (r) mesialsten Punktes sind.

Zusätzlich oder anstelle der Bestimmung des Transversalmaßes erfolgt in bevorzugter Ausführungsform ein Bestimmen der skelettalen Klassen, das heißt insbesondere ein Bestimmen, ob ein Vorbiss oder eine Überbiss vorliegt. Zur Ermittlung der skelettalen Klasse ist es bevorzugt zusätzlich zu den mesial gelegenen Punkten des retromolaren Dreiecks die tiefsten Punkte des Mundhöhlenvorhofs auf der rechten und linken Seite neben dem Lippenbändchen des Unterkiefers zu bestimmen und als zusätzlich Basispunkte zu definieren.

Insbesondere mit Hilfe dieser Basispunkte erfolgt ein Bestimmen eines Bisswinkels $k_c$ und eine Bisshöhe h, die sodann zur Ermittlung der skelettalen Klasse genutzt wird.

Hierbei kann zur Ermittlung des Bisswinkels $k_c$ zunächst eine Mittellinie zwischen den beiden definierten mesial gelegenen Punkten und den beiden tiefsten Punkten des Mundhöhlenvorhofs bestimmt werden. Die zweite Linie wird sodann durch eine Verbindungslinie zwischen dem Mittelpunkt der beiden tiefsten Punkte der Mundhöhlenvorhöfe und einem Punkt am Oberkiefer, dem Punkt Papilla Incisiva definiert.

Der Absatz zwischen dem mittleren Punkt zwischen den beiden tiefsten Punkten der Mundhöhlenvorhöfe und dem Punkt Papilla Incisiva definiert ferner die Bisshöhe h.

Zur mathematischen Bestimmung werden die Koordinaten der beiden mesialen Punkte rdr und rdl sowie der beiden tiefsten Punkte des Mundhöhlenvorhofs mvr und mvl bestimmt zu:

$$rdr\ (x_{rdr};\ y_{rdr};\ z_{rdr})$$

$$rdl\ (x_{rdl};\ y_{rdl};\ z_{rdl})$$

$$mvr\ (x_{mvr};\ y_{mvr}; z_{mvr})$$

$$mvl\ (x_{mvl};\ y_{mvl};\ z_{mvl}) \qquad \text{(Gl. 3)}$$

wobei x, y, z die Koordinaten und r rechts sowie l links bezeichnet.

Die gesuchten Mittelpunkte

$$rdm\ (x_{rdm};\ y_{rdm},\ z_{rdm})$$

$$mvm\ (y_{mvm};\ y_{mvm};\ z_{mvm}) \qquad (Gl.\ 4)$$

berechnen sich aus

$$rdm\left(\frac{x_{rdl}+x_{rdr}}{2};\frac{y_{rdl}+y_{rdr}}{2};\frac{z_{rdl}+z_{rdr}}{2}\right)$$

$$mvm\left(\frac{x_{mvl}+x_{mvr}}{2};\frac{y_{mvl}+y_{mvr}}{2};\frac{z_{mvl}+z_{mvr}}{2}\right) \qquad (Gl.\ 5)$$

Ferner wurden die Koordinaten des Punktes Papilla Incisiva pi ($x_{pi}$; $y_{pi}$; $z_{pi}$) bestimmt. Hieraus kann die Bisshöhe h sowie der Bisswinkel $k_c$ berechnet werden durch

$$h\ (mvm;\ pi) = \sqrt{(x_{mvm}-x_{pi})^2+(y_{mvm}-y_{pi})^2+(z_{mvm}-z_{pi})^2}$$

$$a\ (rdm;\ pi) = \sqrt{(x_{rdm}-x_{pi})^2+(y_{rdm}-y_{pi})^2+(z_{rdm}-z_{pi})^2}$$

$$b\ (rdm;\ mvm) = \sqrt{(x_{rdm}-x_{mvm})^2+(y_{rdm}-y_{mvm})^2+(z_{rvm}-z_{mvm})^2}$$

$$kc = arccos\left(\frac{a^2-b^2-h^2}{-2bh}\right) \qquad (Gl.\ 6)$$

Auf Basis der erhaltenen Werte für den Bisswinkel kc und die Bisshöhe h kann automatisch eine skelettale Klasse festgelegt werden. Dies erfolgt insbesondere durch hinterlegte Grenzwerte. Beispielsweise liegt von der skelettalen Klasse vor:

$$Normalbiss\ (Klasse1):\ kc > 50°\ und \leq 90°$$

$$Überbiss\ (Klasse\ 2):\ kc > 90°$$

$$Vorbiss\ (Klasse\ 3):\ kc \leq 50° \qquad (Gl.\ 7)$$

Selbstverständlich ist es auch möglich, Zwischenklassen zu definieren, um eine weitere Verfeinerung des erfindungsgemäßen Verfahrens zu realisieren. Zur automatischen Bestimmung der Bissart werden zwei interalveoläre Verbindungswinkel $\alpha$ beziehungsweise $\beta$ bestimmt. Der auf der linken Seite des Unterkiefers zu bestimmende Winkel $\alpha$ kann berechnet werden aus

$$w\ (rdl;\ tl) = \sqrt{(x_{rdl}-x_{tl})^2+(y_{rdl}-y_{tl})^2+(z_{rdl}-z_{tl})^2}$$

$$q\ (tl;\ rdr) = \sqrt{(x_{tl}-x_{rdr})^2+(y_{tl}-y_{rdr})^2+(z_{tl}-z_{rdr})^2}$$

$$dr\ (rdr;\ rdl) = \sqrt{(x_{rdr}-x_{rdl})^2+(y_{rdr}-y_{rdl})^2+(z_{rdr}-z_{rdl})^2}$$

$$\alpha = arccos\left(\frac{q^2-w^2-dr^2}{-2wdr}\right) \qquad (Gl.\ 8)$$

wobei

w der Abstand zwischen dem linken am weitesten mesial gelegenen Punkt und dem linken Punkt Tuber Maxillae ist.

Der Winkel $\beta$ auf der rechten Seite kann bestimmt werden durch

$$v\,(rdr; tr) = \sqrt{(x_{rdr} - x_{tr})^2 + (y_{rdr} - y_{tr})^2 + (z_{rdr} - z_{tr})^2}$$

$$p\,(tr; rdl) = \sqrt{(x_{tr} - x_{rdl})^2 + (y_{tr} - y_{rdl})^2 + (z_{tr} - z_{rdl})^2}$$

$$dr\,(rdr; rdl) = \sqrt{(x_{rdr} - x_{rdl})^2 + (y_{rdr} - y_{rdl})^2 + (z_{rdr} - z_{rdl})^2}$$

$$\beta = \arccos\left(\frac{q^2 - v^2 - dr^2}{-2vdr}\right) \qquad\qquad \text{(Gl. 9)}$$

wobei

v der Abstand zwischen dem rechten am weitesten mesial gelegenen Punkt und dem rechten Punkt Tuber Maxillae ist.

Als Bissart ergibt sich aus den Berechnungen sodann

Normalbiss: α und β ≥ 80°

Beidseitiger Kreuzbiss: α und β < 80°

Einseitiger Kreuzbiss: α oder β < 80°               (Gl. 10)

In besonderes bevorzugter Ausführungsform der Erfindung erfolgt somit auf Basis des ermittelten Transversalmaßes, der ermittelten skelettalen Klasse und der ermittelten Bissart durch das System ein Vorschlag einer Auswahl für eine passende Zahnstellung. Aus einer großen Anzahl von Zahnaufstellungen, die 10 bis 20 Zahnaufstellungen umfassen kann, wird dem Zahntechniker oder Zahnarzt mit Hilfe des erfindungsgemäßen Verfahrens eine Auswahl von maximal 2 bis 5 Aufstellungen angeboten, die sich maßgeblich durch ästhetische Formabweichungen in der Front unterscheiden.

Eine derart geringe Anzahl von möglichen Aufstellungen erfolgt einerseits durch die automatische Berechnung des Transversalmaßes, der skelettalen Klasse und der Bissart, sowie insbesondere auch aufgrund manueller Auswahlen. Insbesondere erfolgt eine manuelle Auswahl eines Okklusionskonzepts und/oder einer Zahnlinie und/oder einer Zahnform.

Bei dem Okklusionskonzept kann der Anwender vorzugsweise aus mehreren Okklusionskonzepten auswählen. Hierbei handelt es sich insbesondere um eine Auswahl nach:

- Aufstellung über Bukkalkontakte
- Aufstellung lingualisiert
- Aufstellung nach Prof. Dr. Dr. Grunert
- Aufstellung nach Prof. Dr. Gerber

In bevorzugter Ausführungsform werden dem Benutzer für die Auswahl der Zahnlinie ebenfalls unterschiedliche Zahnlinienkombinationen angeboten.

Hierbei handelt es sich beispielsweise um:

- VITAPAN PLUS® (Frontzähne) und VITA LINGOFORM® (Seitenzähne)
- VITAPAN EXCELL® (Frontzähne) und VITA LINGOFORM® (Seitenzähne)
- - VITA VIONIC® (Front- und Seitenzähne)

Vorzugsweise wird dem Benutzer auch bei der Zahnform eine Auswahl angeboten. Hierbei handelt es sich beispielsweise um:

- Form R (Rechteckig)
- Form O (Schaufelförmig)
- Form T (Dreieckig)

Bei einer weiteren bevorzugten Ausführungsform der Erfindung, durch die die Anzahl und/oder die Qualität der nach der Berechnung angebotenen Aufstellung verringert bzw. verbessert werden kann, erfolgt das Auswählen einer Frontzahngarnitur auf Basis der Nasenbreite. Hierbei ist es ausreichend die insbesondere ausreichend die Nasenbreite ebenfalls in das System einzugeben. Hierbei wird die Nasenbreite gegebenenfalls mit einem Faktor multipliziert. Der Faktor liegt vorzugsweise im Bereich von ca. +/- 2mm Nachstehend wird die Erfindung anhand der anliegenden Zeichnungen bezüglich einer bevorzugten Ausführungsform näher erläutert.

Es zeigen:

Fig. 1 ein schematisches Ablaufdiagramm für die Auswahl einer bestmöglichen Aufstellung für den Patients unter Zuhilfenahme des erfindungsgemäßen Verfahrens,

Fig. 2 eine Übersicht der Filterkriterien,

Fig. 3 eine schematische Ansicht eines digitalisierten Unterkiefers mit den zu bestimmenden Basispunkten,

Fig. 4 eine schematische Ansicht eines digitalisierten Oberkiefer mit den zu bestimmenden Basispunkten,

Fig. 5 eine schematische Ansicht des digitalen Unterkiefers zur Bestimmung des Transversalmaßes,

Fig. 6 eine Tabelle sowie eine Skizze zur Ermittlung des Transversalmaßes,

Fig. 7 eine schematische digitale Ansicht des Unterkiefers zur Ermittlung der skelettalen Klasse,

Fig. 8 eine schematische Seitenansicht des digitalisierten Unter- und Oberkiefers zur Ermittlung der Bisshöhe h und des Bisswinkels $k_c$,

Fig. 9 eine schematische Rückansicht des digitalisierten Ober- und Unterkiefers zur Ermittlung der Bissart,

Fig. 10 eine schematische Ansicht zur Ermittlung des Garniturmaßes und

Fig. 11 und 12 Beispiele von mit dem erfindungsgemäßen Verfahren bestimmten Aufstellungen.

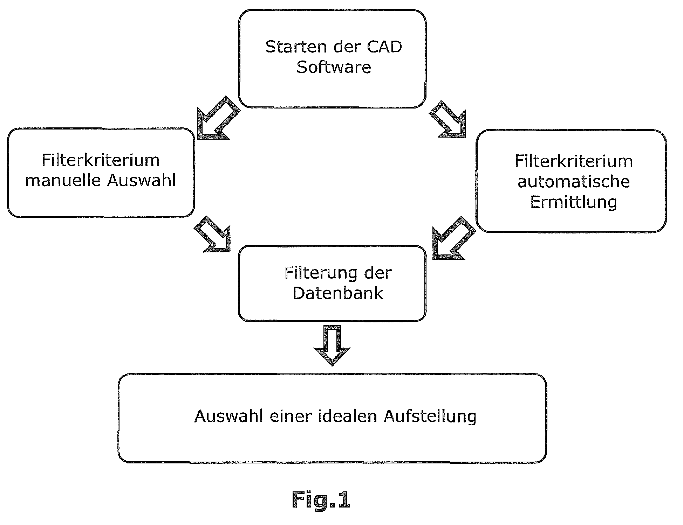

In Fig. 1 ist schematisch der wesentliche Ablauf zur Auswahl einer idealen Zahnaufstellung dargestellt. In einem ersten Schritt wird die Software, bei der es sich insbesondere um eine CAD Software handelt gestartet. Hierbei erfolgt insbesondere auch das Einlesen der digitalisierten oralen Patientensituation, wobei dies selbstverständlich auch zu einem früheren Zeitpunkt bereits erfolgt sein kann. In den nächsten Schritten erfolgt einerseits eine manuelle Auswahl von Filterkriterien und andererseits eine automatische Ermittlung von Filterkriterien auf Grundlage von Berechnungen. Sämtliche Filterkriterien werden sodann im nächsten Schritt zur Filterung der Datenbank, in der die Vielzahl von Zahnaufstellungen hinterlegt ist, genutzt. Hieraus ergibt sich eine Auswahl von nur wenigen geeigneten Zahnaufstellungen, aus denen sodann insbesondere der Zahntechniker auswählen kann.

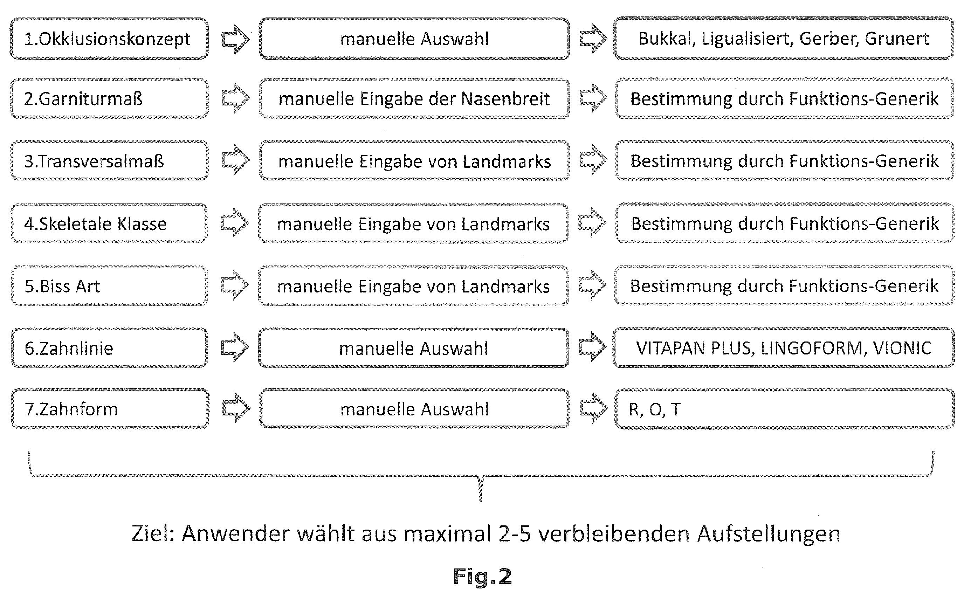

Fig. 2 zeigt eine Übersicht der Filterkriterien. Hierbei sind die mit 1, 6 und 7 bezeichneten Filterkriterien manuell auszuwählende Filterkriterien. Hierbei handelt es sich um das Okklusionskonzept, die Zahnlinie und die Zahnform, wobei auf der rechten Seite der Fig. 2 Beispiele möglicher, für die Benutzer angebotener Auswahlen angegeben sind.

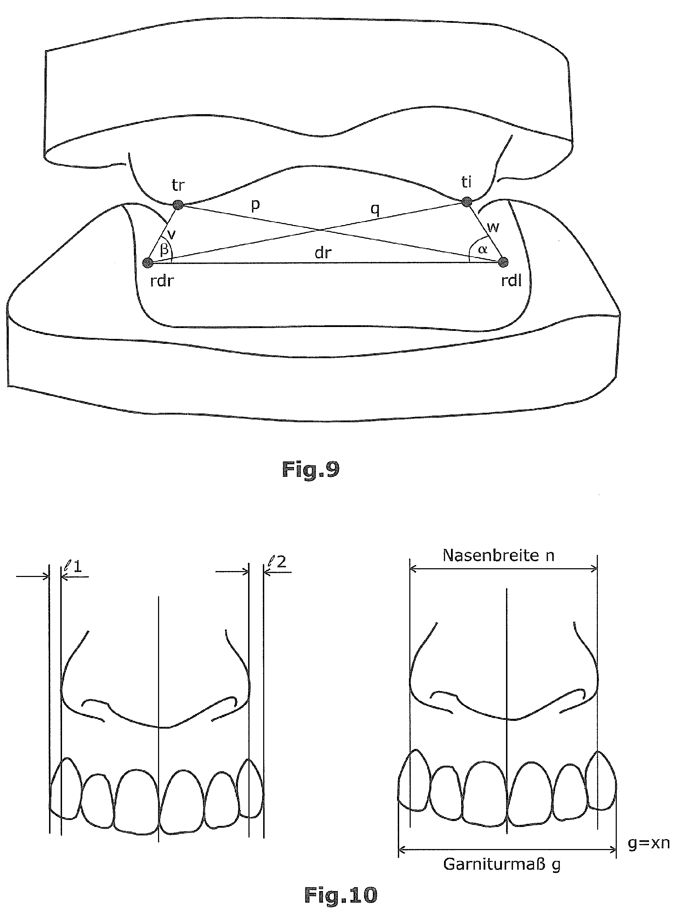

Für die Bestimmung des Garniturmaßes erfolgt vom Benutzer eine manuelle Eingabe der Nasenbreite, wie später an Fig. 10 erläutert.

Zur Bestimmung des Transversalmaßes, der skelektalen Klasse und der Bissart werden unterschiedliche Basispunkte bzw. Landmarks manuell eingegeben oder vom System automatisch ermittelt. Mit Hilfe der hinterlegten Algorithmen werden die entsprechenden Kriterien automatisch bestimmt.

Die Kombination sämtlicher Filterkriterien erfolgt zur Auswahl der idealen Aufstellung anhand der in einer Datenbank hinterlegten großen Anzahl unterschiedlicher Aufstellungen.

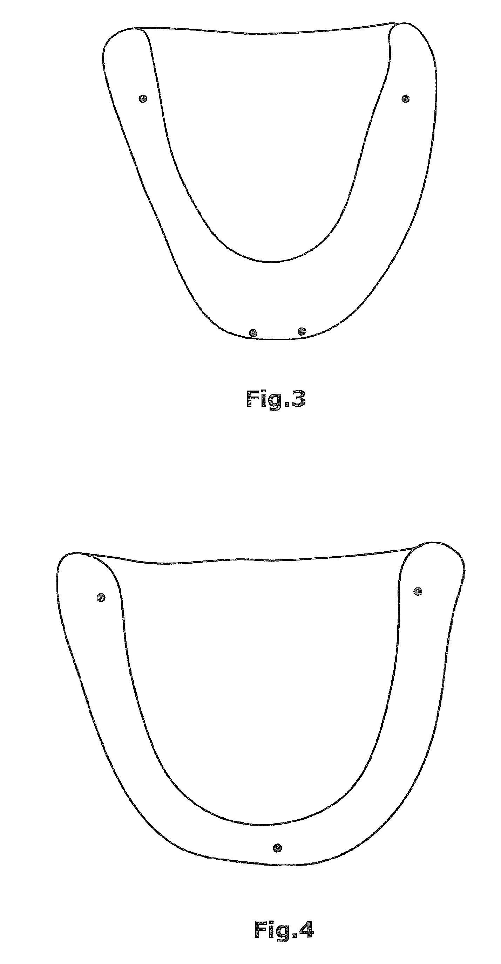

Für die einzelnen durchzuführenden Berechnungen werden Basispunkte bzw. Landmarks definiert. Hierzu wird der Unterkiefer wie in Fig. 3 dargestellt digitalisiert im Rechner abgebildet. Als Basispunkte werden die beiden mesial gelegenen Punkte rdl und rdr auf der linken (l) bzw. auf der rechten Seite (r) des Unterkiefers definiert.

Ferner werden die beiden tiefsten Punkte mvr und mvl des Mundhöhlenvorhofs auf der rechten und linken Seite neben dem Lippenbändchen definiert.

Da der Oberkiefer ebenfalls in digitalisierter Form in dem Rechner abgebildet ist (Fig. 4), erfolgt ferner die Definition des linken und rechten Punktes Tuber Maxillae (tr, tl). Ferner wird im Wesentlichen auf der dem Lippenbändchen des Unterkiefers gegenüberliegenden Seite des Oberkiefers der Punkt Papilla Incisiva (pi) ermittelt.

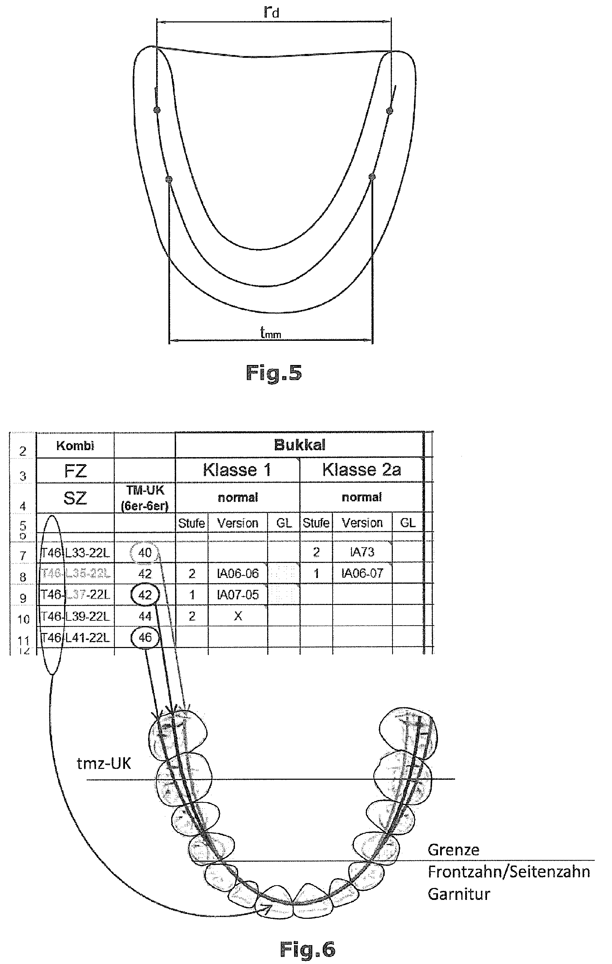

Zur Ermittlung des Transversalmaßes wird, wie in Fig. 5 dargestellt, der Abstand $d_r$ ermittelt. Bei diesem handelt es sich um den Abstand zwischen den beiden mesialen Punkten rdr und rdl. Der Abstand $r_d$ wird sodann mit einem Faktor y multipliziert um gemäß Gleichung 1 das Transversalmaß $t_{mm}$ zu erhalten.

Auf Basis des ermittelten Transversalmaßes kann, wie in Fig. 6 beispielhaft dargestellt eine Aufstellung ermittelt werden. Hierbei können Aufstellungen mit gleicher Frontzahngröße unterschiedliche Zahnbogenmaße aufweisen. Im dargestellten Beispiel wird über das Filterkriterium das Transversalmaß der Aufstellung $t_{ma}$, Mittelfissur 36:46 genutzt, um eine passende Aufstellung auszuwählen. $T_{ma}$ muss hierbei innerhalb des ermittelten Transversalmaßes $T_{mm} \pm$ 1,5mm liegen.

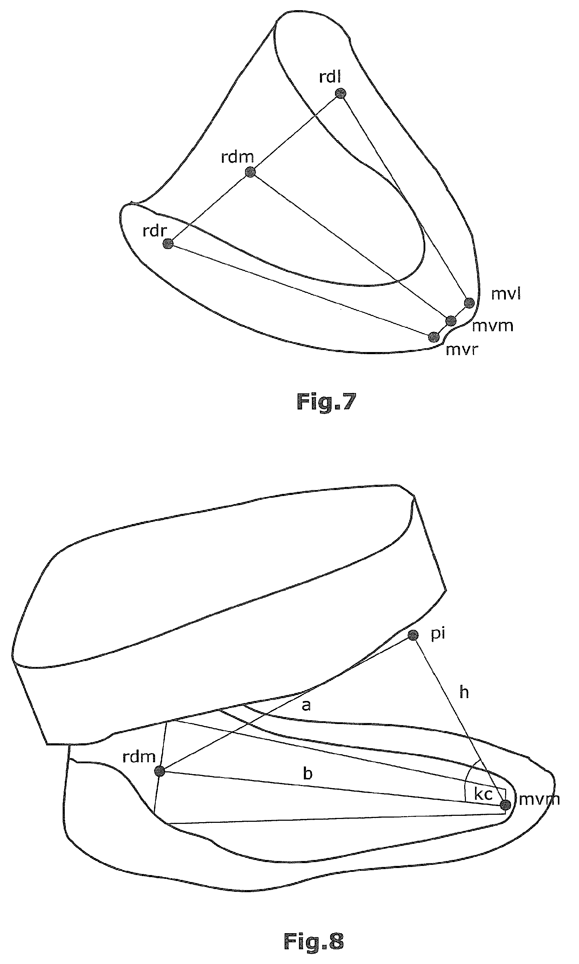

Zur Ermittlung der skelettalen Klasse werden zunächst wie in Fig. 7 dargestellt die beiden definierten mesialen Punkte rdl und rdr miteinander verbunden und der Mittelpunkt rdm zwischen diesen beiden Punkten bestimmt. Entsprechend werden die beiden Punkte im Mundvorhof mvr und mvl miteinander verbunden und wiederum deren Mittelpunkt mvm bestimmt. Diese ergibt eine Verbindungslinie b. Zur Definition einer Verbindunglinie h (Fig. 8) erfolgt ein Verbinden des Punktes mvm mit dem am Oberkiefer definierten Punkt pi (Fig. 4) hieraus ergibt sich der Winkel $k_c$. Des Weiteren wird eine Verbindungslinie a) zwischen den Punkten rdm und pi definiert. Die Berechnung des Winkels erfolgt über die Gleichung 6.

Die Berechnung der Bissart erfolgt anhand der Gleichung 8 und anhand der Gleichung 9 sowie anhand der in Fig. 9 dargestellten Geometrie. Zusätzlich zu dem Abstand $d_r$ zwischen den Punkten rdr und rdl werden die Strecken v zwischen den Punkten rdr und tr sowie w zwischen den Punkten rdl und tl definiert. Des Weiteren erfolgt eine Definition der Strecken p zwischen den Punkten rdl und tr sowie q zwischen den Punkten rdr und tl. Aus den Gleichungen 7 und 8 können sodann die Winkel $\alpha$ und $\beta$ bestimmt werden. Hieraus ergibt sich sodann die Bissart.

Zur Bestimmung der Frontzahngarnitur erfolgt ein Messen der Nasenbreite n (Fig. 10). Zur Nasenbreite n wird l1 + l2 addiert, wobei l1, l2 üblicherweise im Bereich von 0,5 - 3,5 mm liegen. l1 und l2 entsprechen dem Abstand zwischen einer Außenseite des Nasenflügels und einer Außenseite des Eckzahns wie in Fig. 10 dargestellt. Hieraus ergibt sich das Garniturmaß g entsprechend der Gleichung

$$g = l1 + l2 + n \qquad \text{(Gl. 11)}$$

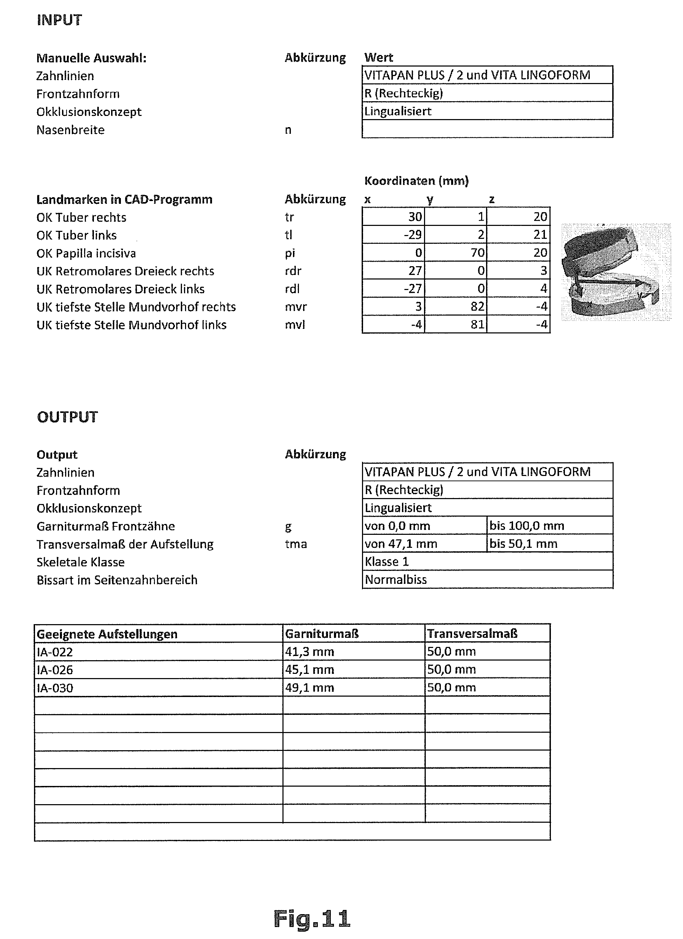

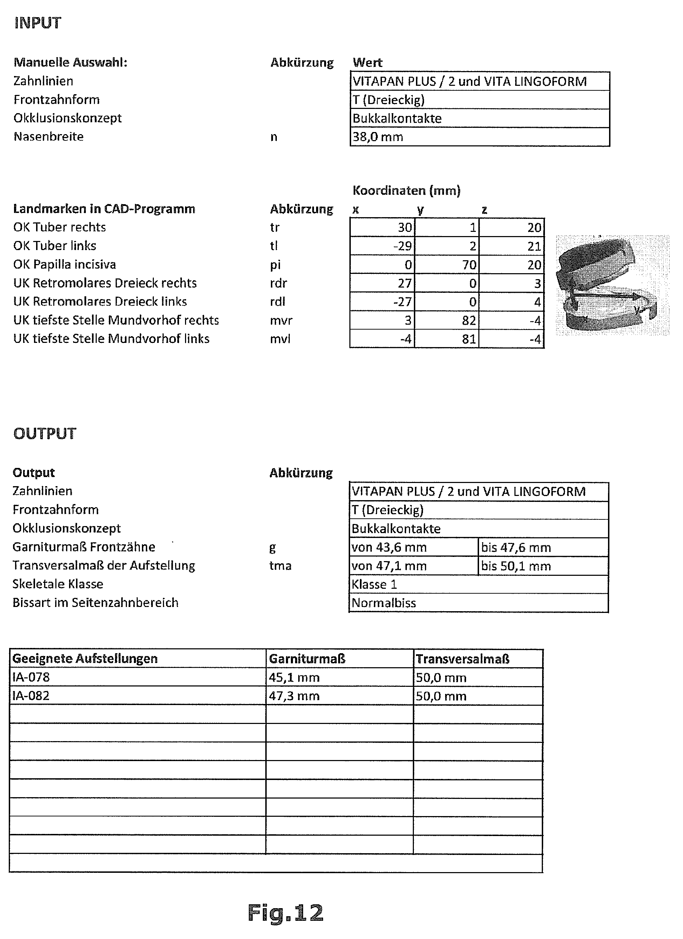

Wie in den Fign. 11 und 12 sind Beispiele für Berechnungen dargestellt. Als manuelle Auswahl bzw. Eingabe erfolgt das Eingeben der Zahnlinie, der Frontzahnform, des Okklusionskonzepts und der Nasenbreite.

Beispiel: Nasenbreite 40mm (Eckzahnmitte zu Eckzahnmitte), Zahnform gemäß Typ O

$$g = 3,0 + 3,0 + 40 \, [mm]$$

$$g = 46,0, \text{ was ungefähr O45 entspricht}$$

Aus den in das CAD Programm eingelesenen bzw. digitalisierten oralen Patientensituationen ergeben sich die Landmarken bzw. Basispunkte mit den entsprechenden Koordinaten. Mit Hilfe erfindungsgemäßen Verfahrens erfolgt sodann ein Filtern von gemäß dem Beispiel in Fig. 11 drei und dem Beispiel in Fig. 12 zwei geeigneten Aufstellungen aus einer Vielzahl in einer Datenbank hinterlegten Aufstellungen.

**Patentansprüche**

1. Verfahren zur Festlegung einer Zahnaufstellung für die Herstellung von Zahnteil- oder Vollprothese mit den Schritten:

   Bestimmen von durch Koordinaten definierte Basispunkte eines Unterkiefers und/oder einer Oberkiefers;
   Ermitteln eines Transversalmaßes und/oder einer skelettalen Klasse und/oder einer Bissart auf Basis der bestimmten Koordinaten für den Unterkiefer und/oder den Oberkiefer und
   Auswahl einer passenden Zahnaufstellung auf Grundlage des ermittelten Transversalmaßes und/oder der ermittelten skelettalen Klasse und/oder der ermittelten Bissart.

2. Verfahren nach Anspruch 1, **dadurch gekennzeichnet, dass** die Basispunkte, die am weitesten mesial gelegenen Punkte (rdl, rdr) der retromolaren Dreiecke auf der rechten und linken Seite des Unterkiefers umfassen.

3. Verfahren nach Anspruch 1 oder 2, **dadurch gekennzeichnet, dass** die Basispunkte, die tiefsten Punkte (mvl, mvr) des Mundhöhlenvorhofs auf der rechten und linken Seite neben dem Lippenbändchen der Unterkieferbasis umfassen.

4. Verfahren nach einem der Ansprüche 1 bis 3, **dadurch gekennzeichnet, dass** die Basispunkte die Punkte (tl, tr) Tuber Maxillae auf der rechten und linken Seite des Oberkiefers umfassen.

**5.** Verfahren nach einem der Ansprüche 1 bis 4, **dadurch gekennzeichnet, dass** die Basispunkte den Punkt (pi) Papilla Incisiva des Oberkiefers umfassen.

**6.** Verfahren nach einem der Ansprüche 2 bis 5, **dadurch gekennzeichnet, dass** das Transversalmaß ($t_{mm}$) zumindest auf Basis des Abstandes ($r_d$) der mesialen Punkte (tl, tr) des retromolaren Dreiecks ermittelt wird.

**7.** Verfahren nach Anspruch 6, **dadurch gekennzeichnet, dass** das Transversalmaß mit einem Korrekturfaktor (y) multipliziert wird.

**8.** Verfahren nach einem der Ansprüche 1 bis 7, **dadurch gekennzeichnet, dass** eine skelettale Klasse auf Basis eines Bisswinkels ($k_c$) und einer Bisshöhe (h) ermittelt wird.

**9.** Verfahren nach Anspruch 8, bei welchem der Bisswinkel ($k_c$) zwischen einer Mittellinie (b), die in der Mitte (mdm, mvm) zwischen den am weitesten mesial gelegenen Punkten (tdl, tdr) und den tiefsten Punkten (mvl, mvr) der Mundhöhlenvorhöfe verläuft und einer Verbindungslinie (h) zu dem Punkt (pi) Papilla Incisiva verläuft.

**10.** Verfahren nach Anspruch 8 oder 9, wobei die Bisshöhe (h) bestimmt wird als Abstand zwischen dem Mittelpunkt mvm der beiden tiefsten Punkte (mvl, mvr) im Mundvorhof und dem Punkt (pi) Papilla Incisiva.

**11.** Verfahren nach einem der Ansprüche 1 bis 10, bei welchem die Bissart auf Basis der interalveolären Verbindungs-winkel ($\alpha$, $\beta$) bestimmt wird.

**12.** Verfahren nach Anspruch 11, bei welchem die Verbindungswinkel ($\alpha$, $\beta$) bestimmt werden durch die Verbindungslinie (v, w, dr) zwischen den beiden am weitesten mesial gelegenen Punkten (tr, tl) des retromolaren Dreiecks und dem linken bzw. rechten Punkt (tr, tl) Tubera.

**13.** Verfahren nach Anspruch 11 oder 12, bei welchem ein Normalbiss bestimmt wird, wenn die beiden Verbindungswinkel ≤80° sind, bei welchem ein beidseitiger Kreuzbiss bestimmt wird, wenn die beiden Verbindungswinkel ≥80° sind und bei welchem einer einseitiger Kreuzbiss bestimmt wird, wenn nur einer der beiden Verbindungswinkel ≥80° ist.

**14.** Verfahren nach einem der Ansprüche 1 bis 13, bei welchem eine Frontzahngarnitur auf Basis einer Nasenbreite n ausgewählt wird, wobei die Nasenbreite (n) gegebenenfalls mit einem Korrekturfaktor (x) multipliziert wird.

**15.** Verfahren nach einem der Ansprüche 1 bis 14, bei welchem zur Auswahl einer Zahnaufstellung für die Unterkieferbasis und/oder die Oberkieferbasis ein Okklusionskonzept und/oder eine Zahnlinie und/oder eine Zahnform aus-gewählt werden.

Starten der CAD
Software

Filterkriterium
manuelle Auswahl

Filterkriterium
automatische
Ermittlung

Filterung der
Datenbank

Auswahl einer idealen Aufstellung

**Fig.1**

| | | |
|---|---|---|
| 1.Okklusionskonzept | ⇨ manuelle Auswahl | ⇨ Bukkal, Ligualisiert, Gerber, Grunert |
| 2.Garniturmaß | ⇨ manuelle Eingabe der Nasenbreit | ⇨ Bestimmung durch Funktions-Generik |
| 3.Transversalmaß | ⇨ manuelle Eingabe von Landmarks | ⇨ Bestimmung durch Funktions-Generik |
| 4.Skeletale Klasse | ⇨ manuelle Eingabe von Landmarks | ⇨ Bestimmung durch Funktions-Generik |
| 5.Biss Art | ⇨ manuelle Eingabe von Landmarks | ⇨ Bestimmung durch Funktions-Generik |
| 6.Zahnlinie | ⇨ manuelle Auswahl | ⇨ VITAPAN PLUS, LINGOFORM, VIONIC |
| 7.Zahnform | ⇨ manuelle Auswahl | ⇨ R, O, T |

Ziel: Anwender wählt aus maximal 2-5 verbleibenden Aufstellungen

**Fig.2**

EP 3 243 481 A1

Fig.3

Fig.4

**Fig.5**

| | Kombi | | Bukkal | | | | | | |
|---|---|---|---|---|---|---|---|---|---|
| | FZ | | Klasse 1 | | | Klasse 2a | | | |
| | SZ | TM-UK (6er-6er) | normal | | | normal | | | |
| | | | Stufe | Version | GL | Stufe | Version | GL |
| | T46-L33-22L | 40 | | | | 2 | IA73 | |
| | T46-L35-22L | 42 | 2 | IA06-06 | | 1 | IA06-07 | |
| | T46-L37-22L | 42 | 1 | IA07-05 | | | | |
| | T46-L39-22L | 44 | 2 | X | | | | |
| | T46-L41-22L | 46 | | | | | | |

tmz-UK

Grenze
Frontzahn/Seitenzahn
Garnitur

**Fig.6**

**Fig.7**

**Fig.8**

**Fig.9**

**Fig.10**

## INPUT

| Manuelle Auswahl: | Abkürzung | Wert |
|---|---|---|
| Zahnlinien | | VITAPAN PLUS / 2 und VITA LINGOFORM |
| Frontzahnform | | R (Rechteckig) |
| Okklusionskonzept | | Lingualisiert |
| Nasenbreite | n | |

**Koordinaten (mm)**

| Landmarken in CAD-Programm | Abkürzung | x | y | z |
|---|---|---|---|---|
| OK Tuber rechts | tr | 30 | 1 | 20 |
| OK Tuber links | tl | -29 | 2 | 21 |
| OK Papilla incisiva | pi | 0 | 70 | 20 |
| UK Retromolares Dreieck rechts | rdr | 27 | 0 | 3 |
| UK Retromolares Dreieck links | rdl | -27 | 0 | 4 |
| UK tiefste Stelle Mundvorhof rechts | mvr | 3 | 82 | -4 |
| UK tiefste Stelle Mundvorhof links | mvl | -4 | 81 | -4 |

## OUTPUT

| Output | Abkürzung | | |
|---|---|---|---|
| Zahnlinien | | VITAPAN PLUS / 2 und VITA LINGOFORM | |
| Frontzahnform | | R (Rechteckig) | |
| Okklusionskonzept | | Lingualisiert | |
| Garniturmaß Frontzähne | g | von 0,0 mm | bis 100,0 mm |
| Transversalmaß der Aufstellung | tma | von 47,1 mm | bis 50,1 mm |
| Skeletale Klasse | | Klasse 1 | |
| Bissart im Seitenzahnbereich | | Normalbiss | |

| Geeignete Aufstellungen | Garniturmaß | Transversalmaß |
|---|---|---|
| IA-022 | 41,3 mm | 50,0 mm |
| IA-026 | 45,1 mm | 50,0 mm |
| IA-030 | 49,1 mm | 50,0 mm |
| | | |
| | | |
| | | |
| | | |
| | | |
| | | |
| | | |
| | | |
| | | |

# Fig.11

15

## INPUT

| Manuelle Auswahl: | Abkürzung | Wert |
|---|---|---|
| Zahnlinien | | VITAPAN PLUS / 2 und VITA LINGOFORM |
| Frontzahnform | | T (Dreieckig) |
| Okklusionskonzept | | Bukkalkontakte |
| Nasenbreite | n | 38,0 mm |

Koordinaten (mm)

| Landmarken in CAD-Programm | Abkürzung | x | y | z |
|---|---|---|---|---|
| OK Tuber rechts | tr | 30 | 1 | 20 |
| OK Tuber links | tl | -29 | 2 | 21 |
| OK Papilla incisiva | pi | 0 | 70 | 20 |
| UK Retromolares Dreieck rechts | rdr | 27 | 0 | 3 |
| UK Retromolares Dreieck links | rdl | -27 | 0 | 4 |
| UK tiefste Stelle Mundvorhof rechts | mvr | 3 | 82 | -4 |
| UK tiefste Stelle Mundvorhof links | mvl | -4 | 81 | -4 |

## OUTPUT

| Output | Abkürzung | | |
|---|---|---|---|
| Zahnlinien | | VITAPAN PLUS / 2 und VITA LINGOFORM | |
| Frontzahnform | | T (Dreieckig) | |
| Okklusionskonzept | | Bukkalkontakte | |
| Garniturmaß Frontzähne | g | von 43,6 mm | bis 47,6 mm |
| Transversalmaß der Aufstellung | tma | von 47,1 mm | bis 50,1 mm |
| Skeletale Klasse | | Klasse 1 | |
| Bissart im Seitenzahnbereich | | Normalbiss | |

| Geeignete Aufstellungen | Garniturmaß | Transversalmaß |
|---|---|---|
| IA-078 | 45,1 mm | 50,0 mm |
| IA-082 | 47,3 mm | 50,0 mm |
| | | |
| | | |
| | | |
| | | |
| | | |
| | | |
| | | |
| | | |
| | | |

## Fig.12

Europäisches
Patentamt

European
Patent Office

Office européen
des brevets

## EUROPÄISCHER RECHERCHENBERICHT

**Nummer der Anmeldung**

EP 16 16 9206

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2010/151417 A1 (NILSSON URBAN [SE] ET AL) 17. Juni 2010 (2010-06-17) <br> * Absatz [0002] * <br> * Absatz [0081] * <br> * Absatz [0097] * <br> * Absatz [0108] * <br> * Absatz [0063] - Absatz [0076] * <br> ----- | 1-15 | INV. <br> A61C9/00 |
| A | WO 2015/169910 A1 (3SHAPE AS [DK]) 12. November 2015 (2015-11-12) <br> * Ansprüche * <br> * Abbildungen * <br> ----- | 1-15 | |
| A | JP 2005 168518 A (OSAKA IND PROMOTION ORG) 30. Juni 2005 (2005-06-30) <br> * das ganze Dokument * <br> ----- | 1-15 | |

**RECHERCHIERTE SACHGEBIETE (IPC)**

A61C

Der vorliegende Recherchenbericht wurde für alle Patentansprüche erstellt

| Recherchenort | Abschlußdatum der Recherche | Prüfer |
|---|---|---|
| München | 26. Oktober 2016 | Fortune, Bruce |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
nach dem Anmeldedatum veröffentlicht worden ist
D : in der Anmeldung angeführtes Dokument
L : aus anderen Gründen angeführtes Dokument

........................................................................................

& : Mitglied der gleichen Patentfamilie, übereinstimmendes
Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 16 16 9206

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

26-10-2016

| Im Recherchenbericht angeführtes Patentdokument | Datum der Veröffentlichung | Mitglied(er) der Patentfamilie | Datum der Veröffentlichung |
|---|---|---|---|
| US 2010151417 A1 | 17-06-2010 | AU 2008256518 A1<br>BR PI0811883 A2<br>CN 102084365 A<br>EP 2156350 A1<br>JP 5427778 B2<br>JP 2010527667 A<br>KR 20100036269 A<br>US 2010151417 A1<br>WO 2008145293 A2 | 04-12-2008<br>18-11-2014<br>01-06-2011<br>24-02-2010<br>26-02-2014<br>19-08-2010<br>07-04-2010<br>17-06-2010<br>04-12-2008 |
| WO 2015169910 A1 | 12-11-2015 | KEINE | |
| JP 2005168518 A | 30-06-2005 | JP 3820403 B2<br>JP 2005168518 A | 13-09-2006<br>30-06-2005 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2011066895 A1
- EP 1864627 A2
- EP 1444965 A2
- WO 2015078701 A